# EUROPÄISCHE PATENTSCHRIFT

(11) **EP 3 421 016 B1**
(45) Veröffentlichungstag und Bekanntmachung des Hinweises auf die Patenterteilung: **13.05.2020**
(21) Anmeldenummer: 18172801.5
(22) Anmeldetag: 17.05.2018
(51) Int. Cl.: A61F 5/01

(54) **DYNAMISCHE HANDORTHESE**
DYNAMIC HAND ORTHOSIS
ORTHÈSE DE MAIN DYNAMIQUE

(30) Priorität: 02.06.2017 DE 102017112260
(43) Veröffentlichungstag der Anmeldung: 02.01.2019
(73) Patentinhaber: Albrecht GmbH, 83071 Stephanskirchen (DE)
(72) Erfinder: Wolf, Michael, 83059 Kolbermoor (DE)
(74) Vertreter: Flach Bauer Stahl Patentanwälte Partnerschaft mbB

(56) Entgegenhaltungen:
- WO-A1-2016/088071
- WO-A2-2006/063347
- DE-A1-102012 002 785
- DE-U1-202015 008 342
- US-A- 5 178 137

## Beschreibung

Die Erfindung betrifft eine dynamische Handorthese gemäß dem Oberbegriff des Anspruches 1.

Dynamische Handorthesen dieser Art werden bei Kontrakturen oder Muskelschwäche der Hand sowie bei Funktionsbeeinträchtigungen der Hand, insbesondere Finger, verwendet, die auf zentrale Ereignisse wie Schädel-Hirn-Trauma oder Schlaganfall zurückzuführen sind. Weiterhin können derartige Handorthesen postoperativ und zum Training der Fingerbeweglichkeit und Fingerkraft sowie bei Schnittverletzungen, Sehnennähten oder Morbus Dupuytren eingesetzt werden.

Eine Handorthese gemäß dem Oberbegriff des Anspruches 1 ist aus der WO 2006/063347 A2 bekannt. Bei dieser bekannten Handschiene werden elastische Federschienen oder Zugbänder verwendet, die auf einem Handschuh längs der Finger geführt sind und die Finger in ihre Extensionsrichtung ziehen. Die Federschienen oder Zugbänder sind an ihrem proximalen Ende an einem Halteschlitten befestigt, der auf einer Handrückenauflageplatte verschiebbar ist und dadurch den beim Beugen der Finger erforderlichen Längenausgleich ermöglicht. Die Handrückenauflageplatte wird von einem manschettenartigen Unterarmhalteabschnitt gehalten, der im Bereich des Handgelenks am Unterarm befestigt werden kann.

Nachteilig ist bei dieser bekannten Handorthese, dass das Beugen und Strecken der Finger nicht auf eine genau definierte Weise erfolgt. Beim Beugen der Finger kann es im Bereich der Fingergelenke zu unangenehmen Druckkräften durch die elastischen Schienen oder Zugbänder kommen. Weiterhin kann es im Bereich des Handschuhs zu Faltenbildung beim Strecken oder Beugen der Finger kommen.

Der Erfindung liegt von daher die Aufgabe zugrunde, eine dynamische Handorthese der eingangs genannten Art zu schaffen, mit der Beuge- und Streckübungen der Finger auf besonders ergonomische, genaue und angenehme Weise durchgeführt werden können.

Diese Aufgabe wird erfindungsgemäß durch eine dynamische Handorthese mit den Merkmalen des Anspruches 1 gelöst. Vorteilhafte Ausführungsformen der Erfindung sind in den weiteren Ansprüchen angegeben.

Die erfindungsgemäße Handorthese umfasst:
- einen zur Befestigung an einem Unterarm ausgebildeten Unterarmhalteabschnitt,
- einen mit dem Unterarmhalteabschnitt verbundenen Handrückenauflageabschnitt, der eine zur Auflage auf dem Handrücken ausgebildete Handrückenauflageplatte umfasst,
- eine zur Aufnahme einer Fingerspitze ausgebildete Fingerkappe,
- eine Fingerkappenhalterung, die ausgebildet ist, sich von der Handrückenauflageplatte längs eines Fingers zur Fingerkappe zu erstrecken, und die ein distales Ende aufweist, an dem die Fingerkappe angeordnet ist,
- ein elastisches Zugband, das proximal im Bereich des Handrückenauflageabschnitts oder Unterarmhalteabschnitts und distal am distalen Ende der Fingerkappenhalterung oder an der Fingerkappe festgelegt ist,
- wobei die Fingerkappenhalterung zumindest überwiegend aus einer Mehrzahl von gelenkig miteinander verbundenen Kettengliedern besteht und in ihrer Längsrichtung verschiebbar auf der Handrückenauflageplatte gelagert ist.

Durch die Erfindung wird eine Handorthese geschaffen, die unter ergonomischen Gesichtspunkten besonders vorteilhaft ist. Die einzelnen Kettenglieder bilden für einen oder für mehrere Finger jeweils eine Gliederkette, deren einzelne Kettenglieder sich um definierte Schwenkachsen gegeneinander verschwenken lassen. Zweckmäßigerweise sind hierbei die Schwenkachsen sämtlicher Kettenglieder einer Gliederkette parallel zueinander ausgerichtet. Da die einzelnen Kettenglieder aus einem steifen Material, insbesondere aus Kunststoff oder Metall, bestehen, hat die Gliederkette auch eine Steifigkeit in seitlicher Richtung, was ein Ausweichen der Gliederkette in seitlicher Richtung verhindert. Eine Faltenbildung beim Beugen und Strecken der Finger wird zuverlässig verhindert. Die erfindungsgemäße Fingerkappenhalterung, das heißt die Gliederkette, liegt ferner nur auf einer Seite, insbesondere an der dorsalen Seite, des Fingers auf und es ist nicht erforderlich, den betreffenden Finger handschuhartig zu umschließen. Auf der der Handfläche zugeordneten Seite des Fingers kann es daher nicht zu Materialstauchungen beim Beugen des Fingers kommen. Besonders vorteilhaft ist ferner, dass die einzelnen Kettenglieder ohne weiteres derart ausgebildet sein können, dass sie auf einfache Weise ausgetauscht werden können. Hierdurch ist es auch auf besonders einfache Weise möglich, die Gliederkette durch Hinzufügen oder Herausnehmen einzelner Kettenglieder zu verlängern oder zu verkürzen und dadurch an unterschiedliche Finger- und Handlängen anzupassen.

Vorteilhafterweise weisen die Kettenglieder einen Schwenkanschlag in Extensions- und/oder Flexionsrichtung auf. Die Extensionsschwenkanschläge können insbesondere derart ausgebildet sein, dass die Gliederkette in ihrer maximalen Extensionsstellung geradlinig in einer Ebene angeordnet ist, die parallel zur Hauptebene der Handrückenauflageplatte liegt oder in dieser angeordnet ist. Die Gliederkette erhält dadurch mittels des Zugbandes eine definierte Ausgangsstellung, die das Anlegen der Handorthese bedeutend erleichtert.

Vorteilhafterweise weisen die Kettenglieder ein Gehäuse mit Gehäusewänden auf, die einen durchgehenden Kanal umschließen. Hierdurch besteht die Möglichkeit, das Zugband sowohl auf den Kettengliedern als auch innerhalb des Kanals zu führen. Hierdurch kann eine sehr exakte, jedoch gleichzeitig enge Führung des Zugbands an den Kettengliedern erreicht werden.

Vorteilhafterweise weisen die Kettenglieder jeweils gleiche Gelenkbolzen und Bolzenaufnahmeöffnungen zur Aufnahme der Gelenkbolzen eines benachbarten Kettenglieds auf. Hierdurch ist es auf besonders einfache Weise möglich, einzelne Kettenglieder zu entfernen oder hinzuzufügen. Besonders vorteilhaft ist es hierbei, wenn die einzelnen Kettenglieder werkzeuglos aneinander befestigt und voneinander gelöst werden können, was insbesondere durch eine Klipp- oder Schnappverbindung zwischen den einzelnen Kettengliedern ermöglicht wird.

Gemäß einer vorteilhaften Ausführungsform umfasst die Gliederkette einen zwischen dem distalen Ende der Handrückenauflageplatte und der Fingerkappe angeordneten Kettenabschnitt mit mindestens zwei Kettengliedern. Dies bedeutet, dass jedem Fingerglied mindestens ein Kettenglied, vorteilhafterweise zwei bis fünf Kettenglieder, zugeordnet wird/werden. Weist die Gliederkette pro Fingerglied nur ein Kettenglied auf, werden die Schwenkachsen zwischen den Kettengliedern zweckmäßigerweise entsprechend der Lage der Fingergelenke positioniert. Für den Daumen erfasst der betreffende Kettenabschnitt somit mindestens zwei Kettenglieder, während für den Zeige-, Mittel-, Ring- und kleinen Finger der betreffende Kettenabschnitt jeweils mindestens drei Kettenglieder, vorteilhafterweise jedoch 6 bis 15 Kettenglieder, umfasst. In diesem Fall kann sich der Kettenabschnitt auf besonders gute Weise der jeweiligen Krümmung des zugeordneten Fingers anpassen.

Gemäß einer vorteilhaften Ausführungsform ist die Fingerkappenhalterung in einem Längsschlitz der Handrückenauflageplatte mittels eines die Handrückenauflageplatte untergreifenden Gleitblocks längsverschiebbar geführt. Dieser Gleitblock kann zweckmäßigerweise einen T-förmigen Querschnitt aufweisen, wobei der Mittelsteg in den Längsschlitz eingreift und damit die Längsführung des Gleitblocks und des daran befestigten Kettenstrangs an der Handrückenauflageplatte bewirkt.

Vorteilhafterweise liegt das elastische Zugband im Bereich zwischen dem distalen Ende der Handrückenauflageplatte und der Fingerkappe auf der Oberseite der Kettenglieder auf und verläuft im Bereich der Handrückenauflageplatte zumindest überwiegend innerhalb des durch die Kettenglieder gebildeten Kanals. Hierdurch kann erreicht werden, dass das Zugband im Bereich der Handrückenauflageplatte nahe an diese herangeführt wird und dort mittels einer Befestigungseinrichtung befestigt werden kann, die nur relativ wenig dorsal über die Handrückenauflageplatte vorsteht. Darüber hinaus ist das Zugband innerhalb des Kanals optimal geschützt und erhält eine perfekte Seitenführung. Alternativ ist es auch ohne weiteres möglich, dass das Zugband längs des gesamten Kettenstrangs auf der Oberseite der Kettenglieder oder innerhalb des durch die Kettenglieder gebildeten Kanals verläuft.

Vorteilhafterweise sind der Handrückenauflageabschnitt und der Unterarmhalteabschnitt mittels einer Halteschiene derart verbunden, dass der Handrückenauflageabschnitt relativ zum Unterarmhalteabschnitt um eine Hochachse schwenkbar und der Abstand zwischen dem Handrückenauflageabschnitt und dem Unterarmhalteabschnitt veränderbar ist. Durch die schwenkbare Befestigung der Handrückenauflageplatte an der Halteschiene kann die Handrückenauflageplatte in Richtung Ulnardeviation bzw. Radialdeviation verschwenkt werden. Ist die gewünschte Schwenkposition erreicht, wird diese mittels einer geeigneten Arretiervorrichtung fixiert.

Vorteilhafterweise ist zwischen dem Handrückenauflageabschnitt und der Halteschiene eine Halte- und Rasteinrichtung zur Verrastung des Handrückenauflageabschnitts in einer oder mehreren bestimmten Schwenkstellungen relativ zum Unterarmhalteabschnitt vorgesehen, wobei die Halte- und Rasteinrichtung mindestens eine Rastscheibe mit einer Verzahnung und ein weiteres Rastelement aufweist, das formschlüssig in die Verzahnung der Rastscheibe eingreift. Vorteilhafterweise besteht auch das weitere Rastelement aus einer Rastscheibe mit einer Verzahnung, die an die Verzahnung der gegenüberliegenden Rastscheibe angepasst ist und in diese eingreift. Vorzugsweise weisen beide Rastscheiben eine Feinverzahnung auf, wodurch eine entsprechend feine Winkelverstellung in Ulnardeviation bzw. Radialdeviation möglich ist. Zweckmäßigerweise sind beide Rastscheiben um eine zentrale Schraube drehbar. Wird diese zentrale Schraube gelöst, kann die Handrückenauflageplatte in Ulnardeviation bzw. in Radialdeviation verschwenkt werden. Durch Festziehen der zentralen Schraube wird die Position fixiert.

Alternativ ist es auch möglich, zwischen Halteschiene und dem Handrückenauflageabschnitt eine Schwenkverbindungseinrichtung vorzusehen, die eine stufenlose und/oder eine federbelastete dynamische Verschwenkung des Handrückenauflageabschnitts relativ zur Halteschiene und zum Unterarmhalteabschnitt ermöglicht.

Vorteilhafterweise ist das elastische Zugband mittels einer lösbaren Klemmeinrichtung auf der Handrückenauflageplatte oder auf dem Unterarmhalteabschnitt festgeklemmt. Durch Lösen dieser Klemmeinrichtung kann das Zugband relativ zur Klemmeinrichtung bewegt werden, wodurch die Zugkraft des Zugbands eingestellt werden kann.

Die Erfindung wird nachfolgend anhand der Zeichnungen beispielhaft näher erläutert. Es zeigen:
- Figur 1:: eine Ansicht schräg von oben einer erfindungsgemäßen Handorthese mit zwei Gliederketten, wobei die Kettenglieder etwas schematisiert dargestellt sind,
- Figur 2:: eine Seitenansicht einer leicht abgeänderten zweiten Ausführungsform einer an eine Hand angelegten erfindungsgemäßen Handorthese,
- Figur 3:: einen mittigen Längsschlitz durch eine Gliederkette im Bereich eines Gleitblocks,
- Figur 4:: eine Ansicht schräg von oben auf ein Kettenglied,
- Figur 5:: eine schematische Darstellung zur Verdeutlichung der Schwenkmöglichkeit der Handrückenauflageplatte in Ulnardeviation und Radialdeviation, und
- Figur 6:: eine räumliche Darstellung eines Gleitblocks in Alleinstellung.

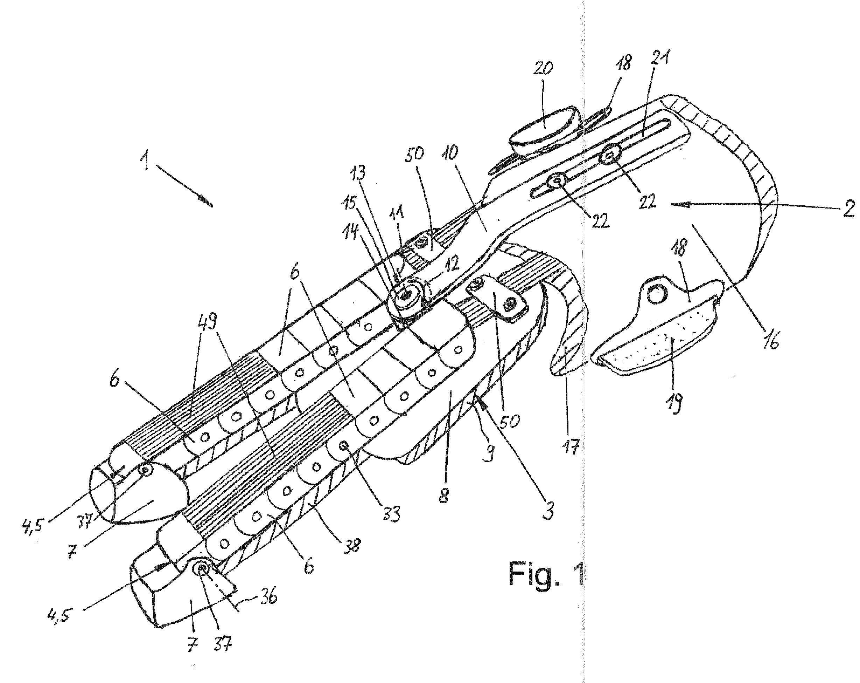

Figur 1 zeigt ein Ausführungsbeispiel einer dynamischen Handorthese 1, die auf zwei Finger eine Zugkraft in Extensionsrichtung, das heißt in Streckrichtung der Finger, aufbringt. Im dargestellten Ausführungsbeispiel handelt es sich bei diesen beiden Fingern, wenn die Handorthese 1 an der rechten Hand angelegt wird, um den Mittel- und Ringfinger.

Die Handorthese 1 weist einen zur Befestigung an einem Unterarm ausgebildeten Unterarmhalteabschnitt 2 und einen mit dem Unterarmhalteabschnitt 2 verbundenen Handrückenauflageabschnitt 3 auf. Vom Handrückenauflageabschnitt 3 erstrecken sich zwei Fingerkappenhalterungen 4 in distaler Richtung über den Handrückenauflageabschnitt 3 hinaus, wobei jede Fingerkappenhalterung 4 aus einer Gliederkette 5 mit einer Mehrzahl von gelenkig miteinander verbundenen Kettengliedern 6 besteht. Am distalen Ende einer jeden Gliederkette 5 ist einen Fingerkappe 7 befestigt, die zum Einführen des vordersten Fingerglieds ausgebildet ist.

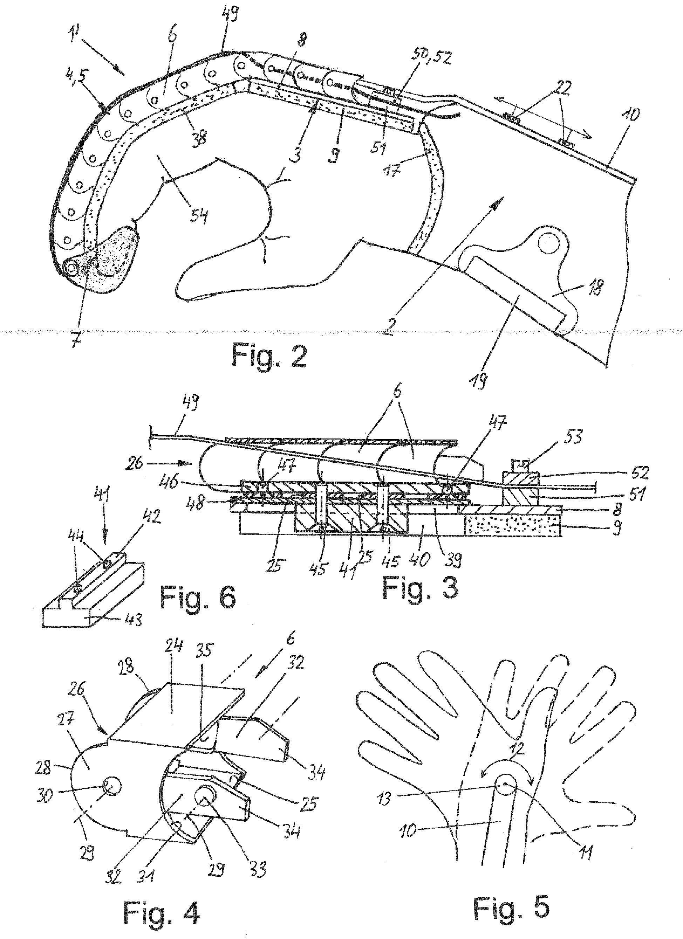

Der Handrückenauflageabschnitt 3 und die Gliederketten 5 sind derart ausgebildet, dass sie von oben her auf den Handrücken bzw. auf den betreffenden Finger aufgelegt werden können, wie aus Figur 2 ersichtlich. Die Ausführungsform der Handorthese 1' von Figur 2 unterscheidet sich von derjenigen von Figur 1 nur darin, dass eine unterschiedliche Anzahl von Kettengliedern 6 vorgesehen und die in Figur 2 dargestellte Ausführungsform mit dem Zeigefinger der rechten Hand zusammenwirkt. Im Übrigen ist jedoch der Aufbau und die Funktionsweise identisch zu der in Figur 1 dargestellten Ausführungsform. Die folgende detaillierte Beschreibung gilt daher sowohl für die in Figur 1 als auch die in Figur 2 dargestellte Ausführungsform.

Der Handrückenauflageabschnitt 3 umfasst eine zur Auflage auf dem Handrücken ausgebildete Handrückenauflageplatte 8, die sich über den größten Teil der Mittelhand erstreckt. Zweckmäßigerweise ist die Handrückenauflageplatte 8 an die Wölbung des Handrückens angepasst. Weiterhin kann die Handrückenauflageplatte 8 beispielsweise aus Metall oder Kunststoff bestehen.

Zur Erhöhung des Tragekomforts ist zweckmäßigerweise auf der Unterseite der Handrückenauflageplatte 8 eine Polsterung 9, beispielsweise in der Form einer Schaumstofflage, befestigt, die auf dem Handrücken aufliegt.

Die Handrückenauflageplatte 8 ist mittels einer Halteschiene 10 am Unterarmhalteabschnitt 2 befestigt. Die Halteschiene 10 ist an ihrem distalen Ende derart gelenkig mit der Handrückenauflageplatte 8 verbunden, dass diese relativ zur Halteschiene 10 und damit relativ zum Unterarmhalteabschnitt 2 um eine Hochachse 11 sowohl in Richtung Ulnardeviation als auch in Richtung Radialdeviation verschwenkt werden kann. Die Hochachse 11 ist somit senkrecht zur Handrückenauflageplatte 8 angeordnet. Die Schwenkmöglichkeit wird durch den Doppelpfeil 12 verdeutlicht und geht auch aus Figur 5 hervor.

Die Verbindung zwischen der Halteschiene 10 und der Handrückenauflageplatte 8 erfolgt über eine Halte- und Rasteinrichtung 13, die in Figur 1 lediglich schematisch dargestellt ist. Diese Halte- und Rasteinrichtung 13 weist eine an der Halteschiene 10 befestigte Rastscheibe 14 und eine auf der Handrückenauflageplatte 8 befestigte weitere Rastscheibe auf, die gleich zur Rastscheibe 14 ausgebildet sein kann und unterhalb dieser angeordnet ist. Die Rastscheibe 14 und die weitere Rastscheibe weisen an ihren einander zugewandten Flächen Verzahnungen auf, die ineinander greifen, wenn die Rastscheibe 14 und die weitere Rastscheibe aufeinanderliegen. Die Rastscheibe 14 und die weitere Rastscheibe werden durch eine Schraube 15 zusammengehalten, welche die Halteschiene 10 und die Rastscheibe 14 durchdringt und in die weitere Rastscheibe eingeschraubt ist. Wird die Schraube 15 etwas gelöst, kann die Rastscheibe 14 von der darunterliegenden weiteren Rastscheibe soweit angehoben werden, dass die beiden Verzahnungen außer Eingriff gelangen und der Handrückenauflageabschnitt 3 relativ zur Halteschiene 10 und zum Unterarmhalteabschnitt 2 verschwenkt werden kann. Wird die Schraube 15 anschließend angezogen, werden die Rastscheiben 14 aneinander gedrückt, so dass die Verzahnungen ineinander greifen und die eingestellte Schwenkposition arretiert ist.

Der Unterarmhalteabschnitt 2 weist eine flexible Schale 16 auf, die derart ausgebildet ist, dass sie den Unterarm in einem handgelenksnahen Bereich zu einem überwiegenden Teil, jedoch nicht vollständig, umschließt. Im gezeigten Ausführungsbeispiel hat die Schale 16 im Wesentlichen eine C-förmige Querschnittsform. Zur Erhöhung des Tragekomforts ist die Schale 16 auf ihrer Innenseite mit einer Polsterung 17, insbesondere aus einem Schaumstoffmaterial, ausgekleidet. Auf den beiden gegenüberliegenden Seiten der Schale 16 ist jeweils eine Haltespange 18 zum Hindurchführen eines Befestigungsbands 19 befestigt. Das Befestigungsband 19 kann durch die beiden Haltespangen 18 hindurchgeführt werden und Klettbereiche aufweisen, um die Länge des Befestigungsbands 19 und damit den Abstand zwischen den beiden Haltespangen 18 einzustellen. Zum einfachen und schnellen Lösen des Befestigungsbandes 19 kann eine der Haltespangen 18 mittels einer Schnalle 20 lösbar an der Schale 16 befestigt sein.

Die Halteschiene 10 ist derart am Unterarmhalteabschnitt 2 befestigt, dass der Abstand zwischen dem Unterarmhalteabschnitt 2 und dem Handrückenauflageabschnitt 3 verändert werden kann. Hierzu weist die Halteschiene 10 in demjenigen Abschnitt, der auf der Schale 16 aufliegt, einen Längsschlitz 21 auf. Durch diesen Längsschlitz 21 sind zwei zueinander beabstandete Schrauben 22, hindurchgeführt, die in die Schale 16 eingeschraubt sind. Die Schraubenköpfe dieser Schrauben 22 übergreifen dabei die Halteschiene 10 und verhindern deren Abheben von der Schale 16. Werden die Schrauben 22 gelöst, kann die Schale 16 relativ zur Halteschiene 10 verschoben werden. Durch Festziehen der Schrauben 22 wird die gewünschte Position fixiert.

Im Folgenden wird die durch eine Gliederkette 5 gebildete Fingerkappenhalterung 4 näher beschrieben. Die Anzahl der Gliederketten kann in Abhängigkeit der Finger, mit denen die Handorthese 1, 1' zusammenwirken soll, variieren und von eins bis fünf betragen. Bei dem in Figur 1 dargestellten Ausführungsbeispiel sind die beiden Gliederketten 5 identisch aufgebaut und unterscheiden sich nur in der Anzahl der Kettenglieder 6. Die längere Gliederkette 5 umfasst im dargestellten Ausführungsbeispiel 10 Kettenglieder 6, während die kürzere Gliederkette 5 neun Kettenglieder 6 umfasst. Die Anzahl der Kettenglieder 6 ist jedoch in weitem Umfang variabel. Weiterhin sind die einzelnen Kettenglieder 6 zumindest überwiegend identisch ausgebildet.

Figur 4 zeigt ein einzelnes Kettenglied 6 in Alleinstellung. Zweckmäßigerweise besteht das Kettenglied 6 aus Kunststoff, kann jedoch auch aus Metall bestehen. Das Kettenglied 6 weist ein Gehäuse mit zwei Seitenwänden 23 auf, die durch eine Deckenwand 24 und eine Bodenwand 25 miteinander verbunden sind. Durch die Seitenwände 23, die Deckenwand 24 und die Bodenwand 25 wird eine Gehäusehülse gebildet, die einen in Längsrichtung des Kettenglieds 6 durchgehenden Kanal 26 umschließt.

Im gezeigten Ausführungsbeispiel ist die Deckenwand 24 und die Bodenwand 25 einstückig an die Seitenwände 23 angeformt. Alternativ ist es jedoch auch möglich, die Deckenwand 24 und/oder Bodenwand 25 mittels eines Scharnier- oder Foliengelenks gelenkig an einer der Seitenwände 23 zu befestigen und an der anderen Seitenwand 23 lösbar, beispielsweise mittels einer Schnappverbindung, zu befestigen, um die Deckenwand 24 und/oder Bodenwand 25 aufklappen zu können. Die Deckenwand 24 und Bodenwand 25 sind als ebene Plattenteile ausgebildet.

Die Seitenwände 23 weisen an einem Ende jeweils einen Endabschnitt 27 mit einer kreisbogenförmig geformten Stirnfläche 28 auf. Diese kreisbogenförmige Stirnfläche 28 erstreckt sich im gezeigten Ausführungsbeispiel über etwa 180° um eine querverlaufende Schwenkachse 29 herum, kann sich jedoch auch über einen Bereich erstrecken, der etwas weniger oder mehr als 180° beträgt. Die kreisbogenförmige Stirnfläche 28 erstreckt sich jeweils von der Deckenwand 24 bis zur Bodenwand 25. Der Durchmesser des Kreisbogens entspricht im dargestellten Ausführungsbeispiel dem lichten Abstand zwischen der Deckenwand 24 und der Bodenwand 25, kann jedoch auch anders dimensioniert sein.

Im Mittelpunkt des Kreisbogens weist jede Seitenwand 23 eine kreisrunde Bolzenaufnahmeöffnung 30 auf, die als Schwenklager zwischen zwei gelenkig miteinander verbundenen Kettengliedern 6 dient.

An ihrem anderen Ende weist jede Seitenwand 23 eine kreisbogenförmig geformte Stirnfläche 31 auf, die konkav ausgebildet ist und deren Radius an denjenigen der Stirnfläche 28 des Endabschnitts 27 angepasst ist. Über diese Stirnfläche 31 ragt in Längsrichtung des Kettenglieds 6 ein Fortsatz 32 vor, der einen seitlich nach außen abstehenden Gelenkbolzen 33 trägt. Der Durchmesser des Gelenkbolzens 33 ist an denjenigen der Bolzenaufnahmeöffnung 33 derart angepasst, dass die Gelenkbolzen 33 eines benachbarten Kettenglieds 6 in die Bolzenaufnahmeöffnungen 30 eingreifen können. Jeder Fortsatz 32 ist relativ zur benachbarten Außenfläche der Seitenwände 23 seitlich etwas nach innen versetzt, derart, dass die Endabschnitte 27 eines benachbarten Kettenglieds 6 über die Fortsätze 32 geschoben werden können, bis die Gelenkbolzen 33 in die Bolzenaufnahmeöffnung 33 einrasten. Benachbarte Kettenglieder 6 können somit um zueinander parallele Querachsen, die durch die Schwenkachsen 29 gebildet werden, relativ zueinander verschwenkt werden.

Der Schwenkbereich kann in den gezeigten Ausführungsbeispielen zweckmäßigerweise zumindest in Extensionsrichtung derart begrenzt werden, dass sich die Gliederkette 5 nicht über die in Figur 1 dargestellte geradlinige gestreckte Stellung hinaus in Extensionsrichtung schwenken lässt. Diese Schwenkbereichsbegrenzung wird im dargestellten Ausführungsbeispiel durch Schwenkanschläge 34 bewirkt, die durch die freien Enden der Fortsätze 32 gebildet werden und weg von den Stirnflächen 31 über die Gelenkbolzen 33 hinaus vorstehen. Die Schwenkanschläge 34 greifen in seitliche Vertiefungen 35 eines benachbarten Kettenglieds 6 ein, die sich auf der Innenseite der Seitenwände 23 befinden. Diese Vertiefungen 35 sind so dimensioniert, dass sich die Schwenkanschläge 34 innerhalb des vorbestimmten Schwenkbereichs verschwenken lassen, während der Schwenkanschlag 34 an der Schwenkbereichsgrenze an der oberen oder unteren Stirnwand der seitlichen Vertiefungen 35 anschlägt.

Der Schwenkanschlag kann auf vielfältige andere Weise realisiert werden. Beispielsweise können die Deckenwände 24 so gestaltet sein, dass die Deckenwände 24 benachbarter Kettenglieder 6 aneinander stoßen, wenn sich die Gliederkette 5 in der maximalen Extensionsstellung befindet.

Die Fortsätze 32 sind zweckmäßigerweise derart elastisch, dass sie beim Aufeinanderstecken benachbarter Kettenglieder 6 nach innen ausweichen und durch ihre eigene Elastizität wieder in ihre Ausgangsstellung zurückfedern können, wenn sich die Gelenkbolzen 33 im Bereich der Bolzenaufnahmeöffnungen 30 befinden.

Die Fingerkappe 7 ist am distalen Ende der Gliederkette 5 am letzten Kettenglied 6 schwenkbar befestigt. Die Fingerkappe 7 ist korbartig und derart ausgebildet, dass die Fingerspitze, das heißt das letzte Fingerglied, in die Fingerkappe 7 eingeführt werden kann. Durch die korb- bzw. kappenartige Ausbildung bildet die Fingerkappe 7 gleichzeitig einen Durchrutschanschlag, der verhindert, dass der Finger über die Fingerkappe 7 in distaler Richtung hinaus durchrutschen kann. Die Fingerkappe 7 besteht zweckmäßigerweise aus einem Textil-, Leder- oder einem flexiblen Kunststoffmaterial. Es ist alternativ auch ohne weiteres möglich, dass die Fingerkappe 7 aus einem formstabilen Material besteht. Die Länge der Fingerkappe 7 ist derart bemessen, dass nur das letzte Fingerglied ganz oder teilweise umschlossen ist.

Um ein einfaches Einführen des Fingers und Ausrichten der Fingerkappe 7 zu ermöglichen, ist die Fingerkappe 7 zweckmäßigerweise um eine quer verlaufende Schwenkachse 36 schwenkbar mit dem letzten Kettenglied 6 verbunden. Diese Schwenkachse 36 kann beispielsweise durch eine zwischen den Seitenwänden 23 angeordnete Hülse mit Innengewinde gebildet werden, in die von den beiden Außenseiten her Schrauben 37 von den beiden Außenseiten her durch die Bolzenaufnahmeöffnungen 30 hindurch eingeschraubt werden.

Die Gliederkette 5 kann in demjenigen Bereich, der über die Handrückenauflageplatte 8 übersteht, auf derjenigen Seite, welche dem Finger zugewandt ist, mit einer Polsterung 38, insbesondere aus einem Schaumstoffmaterial, versehen sein.

Die Gliederketten 5 sind längsverschiebbar auf der Handrückenauflageplatte 8 gehalten und geführt. Hierzu weist die Handrückenauflageplatte 8, wie aus Figur 3 ersichtlich, einen Längsschlitz 39 auf, der fluchtend zur Längsachse der zugeordneten Gliederkette 5 angeordnet ist. Die Polsterung 9 weist unterhalb des Längsschlitzes 39 eine längliche Aussparung 40 auf, die jedoch breiter als der Längsschlitz 39 ausgebildet ist. Innerhalb des Längsschlitzes 39 und der Aussparung 40 ist ein Gleitblock 41 (siehe auch Figur 6) längsverschiebbar geführt. Dieser Gleitblock 41 hat einen T-förmigen Querschnitt und weist einen in Längsrichtung verlaufenden Mittelsteg 42 auf, der von der Unterseite her in den Längsschlitz 39 eingreift. Über den Mittelsteg 42 steht eine Basis 43 beidseitig vor, welche die Handrückenauflageplatte 8 neben dem Längsschlitz 39 untergreift. Der Gleitblock 41 weist ferner zwei Durchgangsbohrungen 44 auf, durch die Befestigungsschrauben 45 (Figur 3) hindurchgeführt werden können. Diese Befestigungsschrauben 45 durchdringen Bohrungen in den Bodenwänden 25 zweier Kettenglieder 6 und sind mit einer Gegenplatte 46 verschraubt, die innerhalb des Kanals 26 angeordnet und auf die Bodenwände 25 aufgelegt ist. Die Gegenplatte 46 ist mittels zweier Schrauben 47 mit den Bodenwänden 25 zweier weiterer Kettenglieder 6 fest verbunden.

Aufgrund dieser Anordnung ist es möglich, dass die Gliederkette 5 zusammen mit dem Gleitblock 41 und der Gegenplatte 46 schlittenartig relativ zur Handrückenauflageplatte 8 in Längsrichtung der Gliederkette 5 verschoben werden kann. Zur Reibungsreduzierung ist hierbei zwischen den Kettengliedern 6 und der Handrückenauflageplatte 8 ein Gleitstreifen 48 aus reibungsverminderndem Material, insbesondere Teflon, angeordnet, der an der Unterseite der Bodenwände 25 aufgeklebt sein kann.

Wie weiterhin aus den Figuren 1 bis 3 ersichtlich, weist die Handorthese 1, 1' für jedes Kettenglied 6 ein elastisches Zugband 49 auf. Dieses Zugband 49 ist proximal im Bereich des Handrückenauflageabschnitts 2 und distal am distalen Ende der Fingerkappenhalterung 4, das heißt am letzten Kettenglied 6, festgelegt. Im Bereich des Handrückenauflageabschnitts 3 erfolgt diese Festlegung mittels einer lösbaren Klemmeinrichtung 50. Diese Klemmeinrichtung 50 umfasst einen unteren Klemmsockel 51 und einen oberen Klemmsockel 52 (Figur 3), der mittels zweier Schrauben 53 am unteren Klemmsockel 51 festgeschraubt werden kann. Das Zugband 49 ist zwischen den beiden Klemmschrauben 53 und zwischen dem unteren und oberen Klemmsockel 51, 52 hindurchgeführt. Durch Lösen der Schrauben 53 kann der obere Klemmsockel 52 etwas vom unteren Klemmsockel 51 abgehoben werden, so dass eine Längenverstellung des Zugbands 49 möglich ist. Befindet sich das proximale Ende des Zugbands 49 in der gewünschten Position, wird der obere Klemmsockel 52 festgeschraubt und das Zugband 49 fest eingeklemmt. Alternativ ist es auch möglich, Klemmeinrichtungen für die Zugbänder 49 vorzusehen, die werkzeuglos geöffnet und geschlossen werden können.

Das Zugband 49 kann an seinem distalen Ende am letzten Kettenglied 6 beispielsweise dadurch befestigt werden, dass es eine distale Schlaufe aufweist, welche die Hülse, in welche die Schrauben 37 eigeschraubt werden, umschlingt.

Das elastische Zugband 49 liegt im Bereich zwischen dem distalen Ende der Handrückenauflageplatte 8 und der Fingerkappe 7 auf der Oberseite der Kettenglieder 6, das heißt auf den Deckenwänden 24, auf und verläuft damit zumindest im Bereich zwischen dem distalen Ende der Handrückenauflageplatte 8 und der Fingerkappe 7 oberhalb der Kettenglieder-Schwenkachsen. Im Bereich der Handrückenauflageplatte 8 verläuft das Zugband 49 innerhalb des durch die Kettenglieder 6 gebildeten Kanals 26. Alternativ hierzu ist es jedoch auch ohne weiteres möglich, dass das Zugband 49 über seine gesamte Länge zwischen der Klemmeinrichtung 50 und seinem distalen Ende oberhalb der Kettenglieder 6 oder innerhalb des Kanals 26 verläuft.

Wie aus Figur 2 ersichtlich, liegt die Gliederkette 5 lediglich auf der Oberseite des zugeordneten Fingers 54 auf. Durch das Zugband wird eine Zugkraft zwischen der Klemmeinrichtung 50 und der Fingerkappe 7 erzeugt, welche den Finger in seine Extensionsstellung zieht. Wird der Finger gebeugt, wie in Figur 2 gezeigt, wird das Zugband 49 gedehnt und übt mit zunehmendem Beugungswinkel eine zunehmende Kraft in Gegenrichtung aus. Durch wiederholtes Beugen und Strecken des Fingers können Funktionsdefizite der Hand vermindert oder beseitigt und die Hand- und Unterarmmuskulatur entsprechend trainiert werden. Der beim Beugen und Strecken des Fingers erforderliche Längenausgleich erfolgt dabei automatisch durch Verschieben des Befestigungsabschnitts der Gliederkette 5 relativ zur Handrückenauflageplatte 8.

Im Rahmen der Erfindung ist eine Vielzahl von Variationen möglich. Beispielsweise ist es möglich, das erfindungsgemäße Prinzip auf Handorthesen anzuwenden, welche den oder die Finger nicht in Extensionsrichtung, sondern in Flexionsrichtung zieht, so dass erhöhte Muskelkraft erforderlich ist, um die Finger in Extensionsrichtung zu bewegen. Eine derartige Ausführungsform kann beispielsweise dadurch realisiert werden, dass das Zugband unterhalb der Schwenkachsen der Kettenglieder 6 geführt wird, wodurch die Gliederketten 5 in Flexionsrichtung gezogen werden.

## Patentansprüche

1. Dynamische Handorthese, umfassend:
- einen zur Befestigung an einem Unterarm ausgebildeten Unterarmhalteabschnitt (2),
- einen mit dem Unterarmhalteabschnitt (2) verbundenen Handrückenauflageabschnitt (3), der eine zur Auflage auf den Handrücken ausgebildete Handrückenauflageplatte (8) umfasst,
- eine zur Aufnahme einer Fingerspitze ausgebildete Fingerkappe (7),
- eine Fingerkappenhalterung (4) die ausgebildet ist, sich von der Handrückenauflageplatte (8) längs eines Fingers (54) zur Fingerkappe (7) zu erstrecken, und die ein distales Ende aufweist, an dem die Fingerkappe (7) angeordnet ist,
- ein elastisches Zugband (49), das proximal im Bereich des Handrückenauflageabschnitts (3) oder Unterarmhalteabschnitts (2) und distal am distalen Ende der Fingerkappenhalterung (4) oder an der Fingerkappe (7) festgelegt ist,
**dadurch gekennzeichnet, dass** die Fingerkappenhalterung (4) zumindest überwiegend aus einer Gliederkette (5) mit einer Mehrzahl von gelenkig miteinander verbundenen Kettengliedern (6) besteht und in ihrer Längsrichtung verschiebbar auf der Handrückenauflageplatte (8) gelagert ist.

2. Handorthese nach Anspruch 1, **dadurch gekennzeichnet, dass** die Kettenglieder (6) einen Schwenkanschlag (34) in Extensions- und/oder Flexionsrichtung aufweisen.

3. Handorthese nach einem der vorhergehenden Ansprüche, **dadurch gekennzeichnet dass** die Kettenglieder (6) ein Gehäuse mit Gehäusewänden aufweisen, die einen durchgehenden Kanal (26) umschließen.

4. Handorthese nach einem der vorhergehenden Ansprüche, **dadurch gekennzeichnet, dass** die Kettenglieder (6) jeweils gleiche Gelenkbolzen (33) und Bolzenaufnahmeöffnungen (30) zur Aufnahme der Gelenkbolzen (33) eines benachbarten Kettenglieds (6) aufweisen.

5. Handorthese nach einem der vorhergehenden Ansprüche, **dadurch gekennzeichnet, dass** die Gliederkette (5) einen zwischen dem distalen Ende der Handrückenauflageplatte (8) und der Fingerkappe (7) angeordneten Kettenabschnitt mit mindestens zwei Kettengliedern (6) umfasst.

6. Handorthese nach einem der vorhergehenden Ansprüche, **dadurch gekennzeichnet, dass** die Fingerkappenhalterung (4) in einem Längsschlitz (39) der Handrückenauflageplatte (8) mittels eines die Handrückenauflageplatte (8) untergreifenden Gleitblocks (41) längsverschiebbar geführt ist.

7. Handorthese nach einem der Ansprüche 3 bis 6, **dadurch gekennzeichnet, dass** das elastische Zugband (49) im Bereich zwischen dem distalen Ende der Handrückenauflageplatte (8) und der Fingerkappe (7) auf der Oberseite der Kettenglieder (6) aufliegt und im Bereich der Handrückenauflageplatte (8) zumindest teilweise innerhalb des durch die Kettenglieder (6) gebildeten Kanals (26) verläuft.

8. Handorthese nach einem der vorhergehenden Ansprüche, **dadurch gekennzeichnet, dass** der Handrückenauflageabschnitt (3) und der Unterarmhalteabschnitt (2) mittels einer Halteschiene (10) derart verbunden sind, dass der Handrückenauflageabschnitt (3) relativ zum Unterarmhalteabschnitt (2) um eine Hochachse (11) schwenkbar und der Abstand zwischen dem Handrückenauflageabschnitt (3) und dem Unterarmhalteabschnitt (2) veränderbar ist.

9. Handorthese nach Anspruch 8, **dadurch gekennzeichnet, dass** zwischen dem Handrückenauflageabschnitt (3) und der Halteschiene (10) eine Halte- und Rasteinrichtung (13) zur Verrastung des Handrückenauflageabschnitts (3) in einer oder mehreren bestimmten Schwenkstellungen relativ zum Unterarmhalteabschnitt (2) vorgesehen ist, wobei die Halte- und Rasteinrichtung (13) mindestens eine Rastscheibe (14) mit einer Verzahnung und ein weiteres Rastelement aufweist, das formschlüssig in die Verzahnung der Rastscheibe (14) eingreift.

10. Handorthese nach einem der vorhergehenden Ansprüche, **dadurch gekennzeichnet, dass** das elastische Zugband (49) mittels einer lösbaren Klemmeinrichtung (50) auf der Handrückenauflageplatte (8) oder auf dem Unterarmhalteabschnitt (2) festgeklemmt ist.

## Claims

1. Dynamic hand orthosis, comprising:
- a forearm holding portion (2) designed for attachment to a forearm,
- a hand-back support portion (3) connected to the forearm holding portion (2), which hand-back support portion comprises a hand-back support plate (8) designed to rest on the back of the hand,
- a finger cap (7) designed to hold a fingertip,
- a finger cap holder (4) which is designed to extend from the hand-back support plate (8) along a finger (54) to the finger cap (7), and which has a distal end at which the finger cap is arranged (7),
- an elastic strap (49) which is arranged proximally in the region of the hand-back support portion (3) or forearm holding portion (2) and distally at the distal end of the finger cap holder (4) or on the finger cap (7),
**characterised in that** the finger cap holder (4) consists at least predominantly of a link chain (5) having a plurality of chain links (6) which are connected to one another in an articulated manner and is mounted on the hand-back support plate (8) so as to be displaceable in the longitudinal direction.

2. Hand orthosis according to claim 1, **characterised in that** the chain links (6) have a pivot stop (34) in the extension and/or flexion direction.

3. Hand orthosis according to either of the preceding claims, **characterised in that** the chain links (6) comprise a housing having housing walls which enclose a continuous channel (26).

4. Hand orthosis according to any of the preceding claims, **characterised in that** the chain links (6) each have identical hinge pins (33) and pin receiving openings (30) for receiving the hinge pin (33) of an adjacent chain link (6).

5. Hand orthosis according to any of the preceding claims, **characterised in that** the link chain (5) comprises a chain portion having at least two chain links (6) arranged between the distal end of the hand-back support plate (8) and the finger cap (7).

6. Hand orthosis according to any of the preceding claims, **characterised in that** the finger cap holder (4) is longitudinally displaceably guided in a longitudinal slot (39) of the hand-back support plate (8) by means of a slide block (41) engaging under the hand-back support plate (8).

7. Hand orthosis according to any of claims 3 to 6, **characterised in that** the elastic strap (49) is in the region between the distal end of the hand-back support plate (8) and the finger cap (7) on the top of the chain links (6) and extends in the region of the hand-back support plate (8) at least partially within the channel (26) formed by the chain links (6).

8. Hand orthosis according to any of the preceding claims, **characterised in that** the hand-back support portion (3) and the forearm holding portion (2) are connected by means of a holding strip (10) such that the hand-back support portion (3) can be pivoted about a vertical axis (11) relative to the forearm holding portion (2) and the distance between the hand-back support portion (3) and the forearm holding portion (2) can be changed.

9. Hand orthosis according to claim 8, **characterised in that** a holding and locking device (13) for locking the hand-back support portion (3) in one or more specific pivot positions relative to the forearm holding portion (2) is provided between the hand-back support portion (3) and the holding strip (10), the holding and locking device (13) having at least one locking disc (14) which comprises a toothing and having a further locking element which positively engages in the toothing of the locking disc (14).

10. Hand orthosis according to any of the preceding claims, **characterised in that** the elastic strap (49) is clamped on the hand-back support plate (8) or on the forearm holding portion (2) by means of a releasable clamping device (50).

## Revendications

1. Orthèse de main dynamique comportant
- une portion de retenue d'avant-bras (2) réalisée pour être fixée à un avant-bras,
- une portion d'appui de dos de main (3) reliée à la portion de retenue d'avant-bras (2) et comprenant une plaque d'appui de dos de main (8) réalisée pour s'appuyer sur le dos de la main,
- un capuchon de doigt (7) réalisé pour recevoir le bout d'un doigt,
- un support de capuchon de doigt (4) réalisé pour s'étendre depuis la plaque d'appui de dos de main (8) le long d'un doigt (54) jusqu'au capuchon de doigt (7) et présentant une extrémité distale à laquelle est disposé le capuchon de doigt (7),
- une bande de traction élastique (49) fixée de manière proximale dans la zone de la portion d'appui de dos de main (3) ou de la portion de retenue d'avant-bras (2) et de manière distale à l'extrémité distale du support de capuchon de doigt (4) ou au capuchon de doigt (7),
**caractérisée en ce que**
le support de capuchon de doigt (4) est constitué au moins en majeure partie d'une chaîne à maillons (5) ayant une pluralité de maillons de chaîne (6) reliés entre eux de manière articulée et est monté sur la plaque d'appui de dos de main (8) en étant mobile dans sa direction longitudinale.

2. Orthèse de main selon la revendication 1,
**caractérisée en ce que**
les maillons de chaîne (6) présentent une butée de pivotement (34) en direction d'extension et/ou de flexion.

3. Orthèse de main selon l'une des revendications précédentes,
**caractérisée en ce que**
les maillons de chaîne (6) présentent un boîtier ayant des parois de boîtier qui entourent un canal traversant (26).

4. Orthèse de main selon l'une des revendications précédentes,
**caractérisée en ce que**
les maillons de chaîne (6) présentent chacun des pivots d'articulation identiques (33) et des ouvertures de réception de pivots (30) pour recevoir les pivots d'articulation (33) d'un maillon de chaîne (6) voisin.

5. Orthèse de main selon l'une des revendications précédentes,
**caractérisée en ce que**
la chaîne de maillons (5) comprend une portion de chaîne ayant au moins deux maillons de chaîne (6) et disposée entre l'extrémité distale de la plaque d'appui de dos de main (8) et le capuchon de doigt (7).

6. Orthèse de main selon l'une des revendications précédentes,
**caractérisée en ce que**
le support de capuchon de doigt (4) est guidé de façon mobile en translation longitudinale dans une fente allongée (39) de la plaque d'appui de dos de main (8) au moyen d'une bloc coulissant (41) engageant par le dessous la plaque d'appui de dos de main (8).

7. Orthèse de main selon l'une des revendications 3 à 6,
**caractérisée en ce que**
la bande de traction élastique (49) repose sur la face supérieure des maillons de chaîne (6) dans la zone entre l'extrémité distale de la plaque d'appui de dos de main (8) et le capuchon de doigt (7) et s'étend dans la zone de la plaque d'appui de dos de main (8) au moins partiellement à l'intérieur du canal (26) formé par les maillons de chaîne (6).

8. Orthèse de main selon l'une des revendications précédentes,
**caractérisée en ce que**
la portion d'appui de dos de main (3) et la portion de retenue d'avant-bras (2) sont reliées au moyen d'une attelle de retenue (10) de telle sorte que la portion d'appui de dos de main (3) est mobile en pivotement autour d'un axe vertical (11) par rapport à la portion de retenue d'avant-bras (2) et que la distance entre la portion d'appui de dos de main (3) et la portion de retenue d'avant-bras (2) est variable.

9. Orthèse de main selon la revendication 8,
**caractérisée en ce que**
entre la portion d'appui de dos de main (3) et l'attelle de retenue (10), il est prévu un moyen de retenue et d'enclenchement (13) pour enclencher la portion d'appui de dos de main (3) dans une ou dans plusieurs positions de pivotement déterminées par rapport à la portion de retenue d'avant-bras (2), le moyen de retenue et d'enclenchement (13) comprenant au moins une rondelle d'enclenchement (14) ayant une denture et un autre élément d'enclenchement qui s'engage par coopération de forme dans la denture de la rondelle d'enclenchement (14).

10. Orthèse de main selon l'une des revendications précédentes,
**caractérisée en ce que**
la bande de traction élastique (49) est coincée sur la plaque d'appui de dos de main (8) ou sur la portion de retenue d'avant-bras (2) à l'aide d'un moyen de coincement amovible (50).
